# FASCICULE DE BREVET EUROPEEN

(11) **EP 0 924 185 B1**
(45) Date de publication et mention de la délivrance du brevet: **18.09.2002**
(21) Numéro de dépôt: 98403164.1
(22) Date de dépôt: 15.12.1998
(51) Int. Cl.: C07C 67/03, C07C 69/52

(54) **Procédé de fabrication d'esters de corps gras**
Verfahren zum Herstellen von Fettsäureester
Process for the preparation of fatty acid esters

(30) Priorité: 18.12.1997 FR 9716293
(43) Date de publication de la demande: 23.06.1999
(73) Titulaire: INSTITUT FRANCAIS DU PETROLE, 92500 Rueil Malmaison (FR)
(72) Inventeur: Stern, Robert, 78700 Conflans Sainte Honorine (FR); Hillion, Gérard, 95220 Herblay (FR); Rouxel, Jean-Jacques, 95450 Longuesse (FR)

(56) Documents cités:
- EP-A- 0 391 485
- FR-A- 2 752 242
- GB-A- 795 573

## Description

La présente invention est relative à un nouveau procédé de fabrication d'esters de corps gras particulièrement purs, procédé réalisé en trois étapes principales : une première étape, dans laquelle on fait réagir une huile végétale ou animale avec un alcool monofonctionnel en présence d'un catalyseur hétérogène, de manière à former un ester d'acide gras de cet alcool ; puis on achève la réaction en évaporant l'excès d'alcool engagé et en séparant par exemple par décantation la glycérine formée ; une deuxième étape, dans laquelle on fait réagir, en présence d'un catalyseur hétérogène, les monoglycérides présents dans l'ester brut obtenu dans la première étape avec l'ester de monoalcool formé, de manière à les transformer en produits plus lourds, tels que des diglycérides et des triglycérides et une troisième étape, dans laquelle on procède à l'évaporation de l'ester brut, non refroidi, obtenu après la deuxième étape, le résidu étant recyclé vers la première étape.

Les applications des esters de monoalcools dérivés d'huiles végétales ou animales sont nombreuses. On signalera principalement l'application comme carburants substituts du gazole dans laquelle on peut envisager d'utiliser comme matières premières les huiles habituelles, telles que par exemple l'huile de colza et l'huile de tournesol, mais aussi des huiles contenant une forte proportion d'acides gras saturés liés à la glycérine, telles que par exemple l'huile de palme ou le suif, ceci pour fabriquer des esters à haute pureté. Cependant, dans cette application, la présence de monoglycérides d'acides gras saturés, même à l'état de traces, est particulièrement gênante, car les monoglycérides ont tendance à cristalliser avec le temps et donc à colmater les filtres.

A cet égard, l'invention propose un procédé qui permet d'obtenir des esters à teneur très réduite en monoglycérides, et qui présente de plus l'avantage de permettre de produire une glycérine très pure, qui, sans distillation, peut être directement utilisée dans des applications industrielles ou alimentaires.

Dans l'application des esters comme carburants substituts du gazole, il est en outre souhaitable de pouvoir utiliser comme matières premières des huiles de friture usagées qui, au départ, contiennent en même temps des produits lourds (dimères, trimères) et des acides gras libres. Le procédé de l'invention permet d'obtenir des esters très purs à partir ce telles matières premières.

D'autres applications sont également recherchées, comme celles qui concernent la fabrication de bases d'esters permettant d'obtenir, par hydrogénolyse d'esters, des alcools ou, par dimérisation ou amidation, des dimères et des amides.

Il est connu de fabriquer des esters méthyliques par les voies classiques de la catalyse homogène avec des catalyseurs solubles, comme la soude ou le méthylate de sodium, en faisant réagir une huile neutre et un alcool comme le méthanol (JAOCS 61, 343-348, 1984).

Deux procédés ont été principalement industrialisés.

Le premier consiste à travailler dans des conditions dans lesquelles on obtient une très bonne conversion de l'huile en esters méthyliques. Aussi n'est-il pas nécessaire d'évaporer l'ester pour éliminer les produits secondaires formés, surtout lorsqu'on a affaire à un ester insaturé, dans lequel la majorité des acides gras liés à l'alcool sont insaturés, c'est-à-dire de type acide oléique ou linoléique. Dans ce cas, les traces de monoglycérides présentes restent solubles dans l'ester.

Un deuxième procédé connu consiste à procéder, à la fin de la première étape de transestérification, à l'évaporation ou à la distillation de l'ester. Pour éviter que les monoglycérides présents dans l'ester soient entraînés, on les transforme en di- et/ou triglycérides en laissant réagir le mélange d'esters obtenu, débarrassé de l'alcool ou de la glycérine, à une température supérieure ou égale à 200 °C, en présence d'un catalyseur basique soluble. Dans ces conditions, il se forme avec le catalyseur alcalin des savons, si bien que le résidu de distillation ou d'évaporation n'est recyclable qu'en partie, car une élimination partielle est nécessaire pour ne pas accumuler les dérivés alcalins et les produits lourds (esters de stérols présents en proportions variables selon la nature de l'huile). Un procédé de ce type est décrit dans la demande EP-A- 0 391 485.

On peut ajouter que les deux procédés exigent, pour la purification de la glycérine, des étapes multiples, car cette glycérine est polluée par les sels alcalins ou les alcoolates, si bien que l'installation de purification de la glycérine est presque aussi coûteuse que celle qui permet la fabrication de l'ester.

Des procédés par catalyse hétérogène qui utilisent un lit fixe ont été décrits dans la littérature, dans différents documents de brevets (brevet EP-B-0 198 243, GB-A-795 573, EP-B-0 198 243 et FR-A-2 752 242).

Ces procédés offrent l'avantage de produire des esters et de la glycérine exempts de catalyseur, donc faciles à purifier. Toutefois, il s'avère que pour obtenir une conversion totale de l'huile en ester, il faut généralement travailler en deux étapes, qui comprennent plusieurs opérations ; ainsi, après la première réaction de transestérification, il faut évaporer l'excès d'alcool, éliminer par décantation la glycérine formée, puis réinjecter de l'alcool avec l'ester partiellement converti, réchauffer à nouveau le mélange et, après réaction, recommencer une évaporation d'alcool et une décantation de la glycérine. Pour obtenir une conversion totale en une fois de l'huile en ester, il faudrait un excès d'alcool très important et un temps de séjour très long (VVH très faible). Si on ne veut pas recourir à toutes les opérations décrites précédemment, il semble préférable de distiller ou d'évaporer l'ester après la première étape de transestérification. La distillation est possible sur l'ester brut, mais exige un investissement lourd d'une colonne à plateaux, avec le risque que l'on connaît d'un fond de colonne surchauffé, ainsi qu'une consommation en énergie importante, due principalement à un taux de reflux obligatoire.

Si l'on recourt à une évaporation ou flash avec un dispositif à film tombant, on risque d'entraîner dans l'ester des traces de monoglycérides dont le point d'ébullition est proche de l'ester, en particulier lorsqu'on est en présence d'esters avec des acides en C₁₈ et des monoglycérides d'acides en C₁₆, ce qui est le cas avec au départ certaines huiles riches en acide palmitique.

Il est difficile d'introduire dans l'ester, comme on le fait dans la catalyse homogène, des produits alcalins susceptibles de transformer les monoglycérides en produits plus lourds car, à coup sûr, ces produits alcalins pollueront le catalyseur hétérogène et même les produits fabriqués comme la glycérine.

On a maintenant découvert de façon surprenante que l'on pouvait éviter totalement ces inconvénients en procédant, avant l'évaporation de l'ester, à une étape de transestérification ou d'estérification des monoglycérides avec les esters d'acides gras présents et/ou, respectivement, par ajout d'au moins un acide gras de 6 à 26 atomes de carbone, en utilisant un catalyseur hétérogène, qui peut être de même nature que celui utilisé dans la première étape.

Grâce à cette seconde étape, réalisée avec un catalyseur hétérogène et qui est pratiquement inverse de la réaction initiale de transestérification, on peut recycler sans perte le résidu de l'évaporation, ne pas polluer le catalyseur hétérogène principal, utiliser une technique d'évaporation avec un appareil à film tombant ou toute autre technique de distillation sans reflux et accéder à un ester pur et à une glycérine pure, cette dernière étant déjà produite dans la première étape.

L'objet de l'invention consiste donc en un procédé de fabrication simultanée d'esters et de glycérine de hautes puretés par un procédé utilisant un catalyseur hétérogène avec trois étapes principales, l'une, connue, qui consiste à faire réagir alcool et huile, la seconde, après élimination de l'alcool en excès et de la glycérine formée, à faire réagir les monoglycérides présents dans l'ester obtenu, avec ce même ester, pour réaliser la réaction décrite ci-dessous : 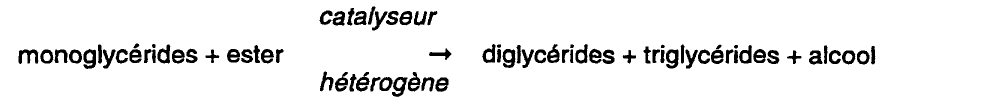 et la troisième étape à évaporer l'ester qui ne contient plus de monoglycéride, cette étape suivant naturellement la seconde, sans qu'il soit nécessaire de refroidir l'ester obtenu dans celle-ci.

Ainsi, d'une manière générale, l'invention propose un procédé de production simultanée d'une composition d'esters d'acides monocarboxyliques linéaires de 6 à 26 atomes de carbone et de glycérine, de hauts degrés de pureté, caractérisé en ce qu'il comprend :
- une étape (a), dans laquelle on fait réagir une huile végétale ou animale, acide ou non, avec un excès d'au moins un monoalcool aliphatique de 1 à 4 atomes de carbone, en présence d'un catalyseur hétérogène choisi parmi les aluminates et les silicates de zinc, de titane ou d'étain, avec élimination de l'alcool en excès et séparation de la glycérine, cette étape produisant un ester brut contenant des monoglycérides résiduels ;
- une étape (b), dans laquelle on soumet l'ester brut ainsi obtenu à une réaction de transestérification ou d'estérification des monoglycérides résiduels en di- et éventuellement triglycérides, en présence d'un catalyseur hétérogène de même nature que celui utilisé à l'étape (a), par les esters d'acides gras ou par au moins un acide gras de 6 à 26 atomes de carbone ajouté ; et
- une étape (c), dans laquelle on procède à une évaporation sous pression réduite de la composition d'esters, avec recyclage du résidu d'évaporation contenant les di- et éventuellement les triglycérides vers l'huile de départ de l'étape (a).

Le procédé de l'invention s'applique à toutes les huiles dont les esters sont faciles à évaporer. Les huiles utilisables sont plus particulièrement des huiles végétales saturées ou insaturées, comme par exemple les huiles de soja, de colza, de tournesol, de lin, d'arachide, de coprah, de palmiste, de palme, de carthame, de linola et de coton, des huiles animales comme le suif, le saindoux et les huiles de poisson, ou des huiles de friture usagées. Toutes ces huiles peuvent être acides, mais les phospholipides en auront de préférence été éliminés avant emploi, par exemple par un procédé connu utilisant l'acide phosphorique ou à l'acide citrique.

On précise ci-après les conditions particulières mises en oeuvre dans chacune des étapes du procédé de l'invention.

Dans le cas où l'on part d'une huile neutre et selon l'alcool engagé, la réaction de l'étape (a) est généralement effectuée à une température comprise entre 180 et 250 °C, de préférence de 180 à 200 °C, à une pression de 20 à 100 bar et une WH (vitesse de passage exprimée en volume d'huile / volume de catalyseur/ heure) de 0,3 à 3. Dans ces conditions, on obtient en général de 85 à 97 % en poids d'esters. Dans cette étape (a), on a intérêt à travailler dans des conditions où l'on obtient la conversion la plus grande, car, dans l'étape (b), on réduit généralement la teneur en ester de 2 à 8 %.

Les catalyseurs hétérogènes utilisés pour réaliser la réaction principale (étape (a)) - comme pour réaliser la réaction secondaire (étape (b)) - sont de préférence les aluminates et, de manière plus préférée, les aluminates de zinc.

Les alcools privilégiés pour la transestérification sont les alcools classiques connus (méthanol, isopropanol, n-butanol, butanol secondaire, isobutanol), mais on utilise de préférence le méthanol si l'on vise la fabrication de substituts du gazole. Dans ce dernier cas, la température est de préférence de 220 à 250 °C et la pression de 40 à 100 bar, plus particulièrement de 30 à 60 bar. Par ailleurs, on opère avec un rapport pondéral méthanol / huile de 1/3 à 2/1.

Le réacteur utilisé pour réaliser l'étape (a) peut être assez court. Pour augmenter le temps de séjour et la vitesse de circulation des réactifs (huile + alcool) sur le catalyseur, il est possible de recycler partiellement une certaine fraction du volume réactionnel sortant du réacteur, tandis que l'autre fraction est envoyée par exemple vers un décanteur - évaporateur, à hauteur en volume du mélange (huile + alcool) qui alimente en continu le réacteur. On peut aussi travailler avec un lit fixe et un simple passage. Après réaction, l'évaporation de l'excès d'alcool (par exemple de méthanol), est réalisée en partie grâce à la détente atmosphérique et, pour les traces d'alcool, par un stripping à l'azote. Après condensation, l'alcool récupéré est sec et peut être recyclé sans qu'il soit nécessaire de le purifier ou de le rectifier, à la condition que l'huile engagée au départ soit neutre et sèche.

La séparation de la glycérine formée dans l'étape (a) se fait en général par décantation à une température allant de 20 à 100 °C.

La glycérine produite dans l'étape (a) est très pure et généralement peu colorée. Si nécessaire, elle peut encore être épurée (décolorée et/ou désodorisée), dans une étape (d), par les voies classiques de purification, comme par exemple par passage sur charbon actif, sur terre activée ou sur un mélange de charbon actif et de terre activée.

De manière préférée, le procédé de l'invention est tel que :
- dans l'étape (a), on fait réagir une huile neutre avec le monoalcool à une température comprise entre 180 et 250 °C, sous une pression de 20 à 100 bar et en présence de 2 à 5 % en poids par rapport à l'huile du catalyseur hétérogène avec élimination par évaporation de l'excès de monoalcool et séparation de la glycérine par décantation à une température de 20 à 100 °C ;
- dans l'étape (b), on transforme les monoglycérides contenus dans l'ester brut en di- et éventuellement triglycérides, le mélange d'esters obtenu contenant moins de 0,2 % de monoglycérides, par chauffage à une température comprise entre 180 et 250 °C, en présence du catalyseur hétérogène et avec élimination du monoalcoolol formé pendant la réaction ; et
- dans l'étape (c), on évapore l'ester de monoalcool formé, sous une pression inférieure ou égale à 3 mm de mercure.

Dans l'étape (b), l'ester brut obtenu dans l'étape (a) peut être introduit en continu soit dans une petite colonne à lit fixe contenant le catalyseur, soit dans un réacteur simple. Dans les deux cas, on élimine continûment ou de façon discontinue l'alcool, par exemple le méthanol, formé. Les temps dépendent de la quantité de catalyseur. Avec de 2 à 5 % en poids de catalyseur par rapport à l'huile, mais on peut en général obtenir en peu de temps une teneur en monoglycérides inférieure à 0,2 %, voire inférieure à 0,1 %, à la fin de l'étape (b).

Les températures préférées pour l'étape (a) et/ou pour l'étape (b) vont de 180 à 200 °C, de manière à éviter le plus possible la formation d'éthers de glycérol.

Par ailleurs, pour éviter de perdre de l'ester, on peut aussi, avant la réaction de l'étape (b), introduire une quantité d'acides gras libres qui réagira préférentiellement avec les monoglycérides à des températures plus basses. En général, on peut introduire un poids égal d'acides gras en C₁₈ par rapport aux monoglycérides. L'estérification est particulièrement rapide avec les catalyseurs à l'aluminate de zinc. Ainsi, on peut par exemple utiliser les acides gras provenant des huiles acides par raffinage. Toutefois, il ne faut pas que ces acides gras contiennent des ions alcalins ou des phospholipides. Cela permet d'une autre manière d'augmenter le rendement en esters par rapport à l'huile mise en jeu. L'autre intérêt de faire réagir des acides gras est que, au lieu de produire de l'alcool, on évapore de l'eau.

On réalise ainsi, à une température à laquelle seul l'acide gras réagit, la réaction bien contrôlée suivante :

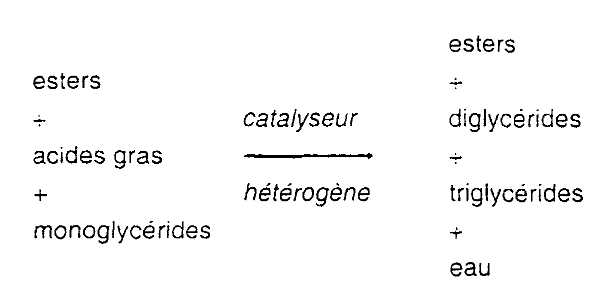

Dans l'étape (c), l'ester, qui ne contient pas ou peu de monoglycérides, est par exemple injecté dans un appareil à film tombant. La pureté de l'ester, si l'on considère seulement la présence de mono-, di- ou triglycérides et d'esters de stérols peut atteindre au moins 99 %. Le vide requis pour cette évaporation est généralement inférieur à 3 mm de mercure avec les esters en C₁₈. L'ester est en général condensé puis stocké et stabilisé au moyen d'un antioxydant.

Si l'huile au départ est acide, par exemple avec un indice d'acide de 10. on peut procéder selon deux voies.

La première consiste à engager directement l'huile acide, qui doit cependant être exempte de phospholipides. On obtient, dans ce cas, une bonne conversion de l'huile, car généralement les acides gras réagissent même mieux que les triglycérides. Toutefois, il se forme de l'eau qui hydrate l'alcool (par exemple le méthanol). La quantité de glycérine formée ne semble pas suffisante pour sécher cet alcool. De plus, cela obligerait à une distillation assez contrôlée de l'alcool (par exemple méthanol), d'où l'obligation de travailler avec un certain taux de reflux.

Si l'alcool est hydraté et si l'on recycle plusieurs fois ce dernier, il se formera à la longue un ester plus ou moins riche en acidité grasse venant de l'hydrolyse de l'ester méthylique. Pour éviter cet ennui, qui à la longue risque d'accumuler l'eau dans l'alcool (méthanol), si l'hydrolyse est incomplète, on procédera selon la seconde voie, qui consiste, avant d'engager l'huile, à réduire sa teneur en acide gras en la faisant réagir avec de faibles quantités de glycérine, en présence d'un catalyseur hétérogène, qui peut être le même que dans la première étape, ou un catalyseur hétérogène similaire.

Comme cela est connu, on obtient ainsi une huile peu ou pas acide dans de bonnes conditions entre 180 et 200 °C, à pression atmosphérique ou sous un léger vide ou par un entraînement à l'azote.

Dans les deux cas précités, où l'huile acide se trouve estérifiée soit avec un monoalcool soit avec de la glycérine, il est préférable d'utiliser un catalyseur hétérogène, défini comme précédemment, mais qui forme difficilement un sel avec un acide gras dans les conditions de réaction.

Dans le procédé de l'invention, pour la réaction principale avec le monoalcool (étape(a)), on a intérêt à utiliser une colonne à lit fixe, car la pression est relativement élevée, par exemple jusqu'à environ 60 ou 100 bar. Pour la réaction des huiles acides avec la glycérine, dans lesquelles on élimine de l'eau, et pour la réaction de transestérification des monoglycérides (étape(b)), dans laquelle on élimine de l'alcool, on peut travailler avec de simples réacteurs en batch, puisque les pressions d'utilisation sont proches de la pression atmosphérique ou en dessous de celle-ci.

Le recyclage des résidus d'évaporation, à la différence du cas des résidus obtenus en catalyse homogène, ne pose aucun problème, car ce résidu ne contient pas de produit nouveau. Une partie des stérols sous forme d'esters de stérols, ainsi que les di- et triglycérides sont présents dans le résidu. Toutefois. lorsque ce résidu ajouté à l'huile est soumis à l'action de l'alcool (en particulier méthanol) en excès, les produits nommés se retransforment en stérols, esters alkyliques (en particulier méthyliques) et en glycérine.

On donne ci-après quelques exemples qui ne sont pas limitatifs.

### Exemple 1

### • Etape 1 :

On fait passer de bas en haut dans un réacteur tubulaire de 0,47 m de long et de 2,7 cm de diamètre, chauffé par 3 coquilles à 230 °C et contenant 70 cm³ d'un catalyseur à base d'aluminate de zinc sous forme d'extrudés, une huile de colza neutre (neutralisée et blanchie) à un débit de 70 cm³/heure et de l'alcool méthylique sec à un même débit. A la sortie du réacteur à lit fixe est connecté un décanteur qui recueille l'ester brut, l'alcool en excès et la glycérine.

Dans le décanteur, lorsqu'un certain niveau est atteint, on procède à un soutirage automatique de la phase liquide. On collecte ainsi l'ester et la glycérine produite sur plusieurs jours.

Le méthanol présent dans l'ester est évaporé en présence de la glycérine dans un évaporateur rotatif de type « Büchi ». La glycérine est ensuite décantée à 50 °C. Elle est purifiée par un traitement combiné de terre activée et de charbon actif à une température de 80 à 90 °C, puis filtrée. Un entraînement à la vapeur peut enfin désodoriser la glycérine ainsi décolorée. Il reste un ester brut, dont la composition est donnée dans le Tableau 1 (première ligne).

### • Etape 2

Cet ester est soumis à une deuxième opération de réestérification des monoglycérides selon le procédé décrit. Dans un ballon en verre de 1 litre, muni d'un agitateur magnétique, d'un chauffage électrique et d'un réfrigérant à reflux avec soutirage possible en tête de colonne du méthanol, on introduit 500 g d'ester brut obtenu dans la première étape, on ajoute 25 g d'un aluminate de zinc broyé équivalent en composition au catalyseur utilisé pour fabriquer l'ester, on chauffe l'appareillage pour obtenir une température maximum de 250°C en fond de ballon. Le temps zéro est considéré comme le temps nécessaire pour atteindre 250°C soit :52 minutes. Un échantillon est prélevé toutes les heures afin de suivre la disparition des monoglycérides et l'augmentation des di- et triglycérides.

L'analyse est réalisée par chromatographie par perméation de gel (CPG). A la fin de la réaction, on filtre le catalyseur. Les résultats de cette étape sont indiqués dans le Tableau 1 où l'on constate qu'après 2 h de réaction, il n'y a plus de monoglycéride présent dans l'ester.

**TABLEAU 1**

| Teneur (en % poids) | TRI | Di | MONO | ESTERS |
|---|---|---|---|---|
| Produit de départ | 2,2 | 2,8 | 4 | 91 |
| T = 0 (après 52 min de chauffage) | 2,8 | 3,6 | 3,3 | 90,3 |
| T = 15 min | 2,9 | 4.3 | 3,1 | 89,7 |
| T = 1 h | 3,6 | 6,7 | 2,0 | 87,7 |
| T = 2 h | 6,2 | 7,9 | - | 85,9 |
| T = 3 h | 6,5 | 6.6 | - | 86,9 |
| TRI = triglycérides Di = diglycérides MONO = monoglycérides ESTERS = esters méthyliques de l'huile de colza | | | | |

### • Etape 3

On distille le filtrat dans un ballon en verre en connectant directement le ballon récepteur par une jonction très courte, suivie d'un condenseur de manière à éviter tout reflux.

La température au fond du ballon est fixée à 250 °C maximum et le vide dynamique est de 2 à 3 mm de mercure. On obtient un distillat qui, analysé par chromatographie par perméation de gel (CPG), n'indique aucune trace de monoglycéride. La méthode d'analyse permet d'avoir une sensibilité de 0,1 à 0,2 %. Pour vérifier l'absence totale de monoglycérides, on pratique une analyse plus poussée qui consiste en une chromatographie en phase vapeur après silylation de l'échantillon.

On constate à nouveau l'absence de monoglycérides.

### Exemple 2 (comparatif)

Pour vérifier la nécessité de travailler en présence de catalyseurs dans l'étape 2, on a chauffé l'ester brut obtenu en 1 selon les conditions de l'exemple 1. mais sans catalyseur. Les résultats sont consignés dans le Tableau 2. On remarque qu'il n'y a pratiquement pas de disparition des monoglycérides.

**TABLEAU 2**

| Teneurs (en % poids) | TRI | Di | MONO | ESTERS |
|---|---|---|---|---|
| Produit de départ | 2,2 | 2,8 | 4,0 | 91 |
| T = 0 | 2,9 | 3,9 | 3,9 | 89,3 |
| T = 1 h | 2,7 | 4,3 | 3,6 | 89,4 |
| T = 2 h | 2,9 | 5,0 | 3,4 | 88,7 |
| T = 3 h | 2,9 | 5,5 | 3,2 | 88,4 |
| T = 5 h | 3,1 | 6,5 | 2,9 | 87,5 |

### Exemple 3 (comparatif)

On évapore selon le même système utilisé dans l'exemple 1 l'ester brut non traité. On obtient un ester qui contient 1,1 % en poids de monoglycérides.

### Exemple 4

On traite une huile de colza acide ayant un indice d'acide de 10,2 obtenu par un ajout d'acide oléique dans une huile de colza raffinée. 200 g de cette huile sont chauffés en présence de 4 g de glycérine et de 4 g d'aluminate de zinc broyé. On maintient une température de 220 °C pendant 5 heures dans un ballon analogue à celui de l'exemple 1 (étape 2), sauf que l'on recueille de l'eau et non de l'alcool. Le mélange obtenu, considéré comme une huile neutralisée est utilisé comme produit de départ dans un procédé tel que celui décrit dans l'exemple 1, en enchaînant les étapes 1, 2 et 3. Le Tableau 3 indique les valeurs d'acidité obtenues après ce traitement.

**TABLEAU 3**

| Teneurs (en mg de KOH/g) | I.A sans azote | I.A avec bullage d'azote |
|---|---|---|
| Produit de départ | 10,5 | 10,3 |
| T = 0 (après 40 min pour atteindre 220 °C ) | 7,4 | 4,4 |
| Après 1 h à 220 °C | 3,4 | 1,7 |
| Après 2h à 220 °C | 2,6 | 1,3 |
| Après 3 h à 220 °C | 2,3 | 0,9 |
| Après 4 h à 220 °C | 2,3 | 0,9 |
| Après 5 h à 220 °C | 2,4 | 0,9 |
| Après 6 h à 220 °C | 2,04 | 0,9 |

### Exemple 5

On fait passer, selon l'exemple 1 (étape 1), une huile de friture dont la composition pondérale est la suivante :
<C16:0 = 3,2
C16:0 = 20,6
C16:1 = 1,6
C17:0 = 0,5
C17:1 = 0,4
C18:0 = 10,9
C18:1 = 39
C18:2 = 20,2
C18:3 = 0,6
>C18 = 0,9
à travers un réacteur tubulaire rempli de catalyseur, sous la forme d'extrudés, à une VVH = 1, soit dans les mêmes conditions que l'huile de colza.

L'indice d'iode de l'huile est de 73,5, son indice d'acide est faible, de l'ordre de 1,2, et la teneur en polymères est de 1,6 % en poids.

Cette huile, après transestérification donne le résultat suivant :
triglycérides = 1,4 %
diglycérides = 2,5 %
monoglycérides = 3,6 %
esters = 92 % (teneurs exprimées en % poids).

Après l'étape 2, on a un produit dont la teneur en monoglycérides est inférieure à 0,1 % mais la teneur en esters n'est plus que de 86 %. La distillation, de type flash, permet d'obtenir un composé d'une pureté supérieure à 99,8 %. Le point d'écoulement de cet ester est voisin de 6 °C.

### Exemple 6

On traite une huile acide (à plus de 32 % en poids d'acides gras) par 6 % de glycérine par rapport à l'huile, en présence de 2,5 % en poids par rapport à l'huile du même catalyseur que celui utilisé dans l'exemple 1, à une température de 220 °C. On obtient, après moins de 2 heures, une huile dont l'indice d'acide est inférieur à 1. Cette huile est traitée dans un autoclave avec du méthanol dans un rapport pondéral alcool/huile = 1. La pression à 230 °C est de 46 bar. On obtient un produit à plus de 96 % d'esters et à 3,1 % de monoglycérides. Après filtration du catalyseur, l'alcool est évaporé d'abord par détente puis dans un évaporateur rotatif. La glycérine est décantée et séparée. L'ester brut est à nouveau soumis à un traitement thermique avec le catalyseur précédent mais sous une pression réduite de 100 mm de mercure. Après évaporation du méthanol à 220 °C on élimine le catalyseur par filtration et on procède sous pression réduite au flash de l'ester brut. Le distillat est exempt de monoglycéride.

### Exemple 7

On traite l'ester brut, obtenu à VVH = 2 avec un catalyseur au titane, obtenu par action d'oxyde de titane sur une alumine; la composition après l'étape 1 est la suivante :
triglycérides = 11.5 %
diglycérides = 7,1 %
monoglycérides = 6,4 %
esters = 75 % (teneurs exprimées en % poids).

On procède à l'étape 2, avec le même catalyseur au titane, à 230 °C et avec 5% de catalyseur, selon la procédure de l'exemple 2. Le Tableau 4 suivant indique les résultats avec et sans catalyseur.

**TABLEAU 4**

| | TRI | | Dl | | MONO | | ESTER | |
|---|---|---|---|---|---|---|---|---|
| Teneurs (en % poids) | avec | sans | avec sans | | avec | sans | avec | sans |
| Produit de départ | 11,5 | | 7,1 | | 6,3 | | 75,1 | |
| Après 15' | 12,5 | 1.2,5 | 8,5 | 7,9 | 4,9 | 5,9 | 74 | 73,5 |
| Après 1 h | 13,4 | 12,5 | 11,1 | 8,2 | 3,05 | 5,9 | 72,2 | 73,4 |
| Après 2 h | 14,4 | 13,8 | 13,2 | 8,9 | 2,0 | 5,6 | 70,1 | 71,7 |
| Après 3 h | 15,0 | 11,3 | 14,5 | 8 | 1,1 | 5,45 | 69,1 | 75,2 |
| Après 4 h | 16,1 | 10,5 | 16 | 8,5 | 0,2 | 5,7 | 67,5 | 75 |

En partant d'un produit contenant 75 % d'esters méthyliques, on réduit sensiblement la teneur en esters dans le produit final (67,5 %), par contre les monoglycérides disparaissent pratiquement complètement.

### Exemple 8

A 500 g d'un mélange d'esters bruts (produit obtenu à VVH = 0,5, avec un rapport volumique huile / méthanol = 1 et en présence d'un catalyseur au zinc), dont la composition la suivante
triglycérides = 0,02 %
diglycérides = 0,10 %
monoglycérides = 3,90 %
esters méthyliques = 95,80 %.
on ajoute 25 g d'acide oléique du commerce.

On obtient, après chauffage à 200 °C pendant 2 heures en utilisant 2 % de catalyseur au zinc, un ester qui ne contient plus que 92 % d'esters. Malgré cette baisse de conversion, la quantité en poids d'esters fabriquée reste finalement la même que celle obtenue lors de la première étape, si l'on ramène cette quantité à 523 g de produit final (la perte de poids d'environ 2 g correspond à l'eau formée pendant l'estérification de l'acide oléique).

Les monoglycérides ont donc été transformés en un mélange de diglycérides et triglycérides jusqu'à hauteur de 8 %. Les monoglycérides et l'acidité grasse ne sont plus présents qu'à l'état de traces.

## Revendications

1. Procédé de production simultanée d'une composition d'esters d'acides monocarboxyliques linéaires de 6 à 26 atomes de carbone et de glycérine, de haut degré de pureté, **caractérisé en ce qu'**il comprend :
- une étape (a), dans laquelle on fait réagir une huile végétale ou animale, acide ou non, avec un excès d'au moins un monoalcool aliphatique de 1 à 4 atomes de carbone, en présence d'un catalyseur hétérogène choisi parmi les aluminates et les silicates de zinc, de titane ou d'étain, avec élimination du monoalcool en excès et séparation de la glycérine, cette étape produisant un ester brut contenant des esters d'acides gras et des monoglycérides résiduels ;
- une étape (b), dans laquelle on soumet l'ester brut ainsi obtenu à une réaction de transestérification ou d'estérification des monoglycérides résiduels en di- et éventuellement triglycérides, en présence d'un catalyseur hétérogène de même nature que celui utilisé à l'étape (a), par les esters d'acides gras ou par au moins un acide gras de 6 à 26 atomes de carbone ajouté ; et
- une étape (c), dans laquelle on procède à une évaporation sous pression réduite de la composition d'esters, avec recyclage du résidu d'évaporation contenant les di- et éventuellement les triglycérides vers l'huile de départ de l'étape (a).

2. Procédé selon la revendication 1 **caractérisé en ce que**, à l'étape (a) et à l'étape (b), on utilise comme catalyseur hétérogène un aluminate.

3. Procédé selon la revendication 2 **caractérisé en ce que** ledit catalyseur hétérogène est l'aluminate de zinc.

4. Procédé selon l'une des revendications 1 à 3 **caractérisé en ce que**
- dans l'étape (a), on fait réagir une huile neutre avec le monoalcool à une température comprise entre 180 et 250 °C, sous une pression de 20 à 100 bar et en présence de 2 à 5 % en poids par rapport à l'huile du catalyseur hétérogène avec élimination par évaporation de l'excès de monoalcool et séparation de la glycérine par décantation à une température de 20 à 100 °C ;
- dans l'étape (b), on transforme les monoglycérides contenus dans l'ester brut en di- et éventuellement triglycérides, le mélange d'esters obtenu contenant moins de 0,2 % de monoglycérides, par chauffage à une température comprise entre 180 et 250 °C, en présence du catalyseur hétérogène et avec élimination du monoalcoolol formé pendant la réaction ; et
- dans l'étape (c), on évapore l'ester de monoalcool formé, sous une pression inférieure ou égale à 3 mm de mercure.

5. Procédé selon l'une des revendications 1 à 4, **caractérisé en ce que** la température des étapes (a) et/ou (b) est de 180 à 200 °C.

6. Procédé selon l'une des revendications 1 à 5, **caractérisé en ce que**, dans une étape (d), on purifie la glycérine formée dans l'étape (a).

7. Procédé selon la revendication 6, **caractérisé en ce que**, dans l'étape (d), on purifie la glycérine par passage sur de la terre activée et/ou du charbon actif.

8. Procédé selon l'une des revendications 1 à 7, **caractérisé en ce que**, au cours de l'étape (b), on ajoute dans l'ester brut issu de l'étape (a) au moins un acide gras ne contenant ni phospholipides, ni ions alcalins, de manière à transformer les monoglycérides en di- et triglycérides avec production d'eau.

9. Procédé selon l'une des revendications 1 à 8, **caractérisé en ce que** l'huile de départ est choisie parmi les huiles de soja, de colza, de toumesol, de lin, d'arachide, de coprah, de palmiste, de palme, de carthame, de linola, de coton, le suif, le saindoux, les huiles de poisson et les huiles de friture usagées.

10. Procédé selon l'uns des revendications 1 à 9, **caractérisé en ce que** le monoalcool aliphatique est choisi parmi le méthanol, l'éthanol, le butanol secondaire et l'isobutanol.

11. Procédé selon la revendication 10, **caractérisé en ce que** ledit monoalcool aliphatique est le méthanol.

12. Procédé selon la revendication 11 **caractérisé en ce que**, dans l'étape (a), on fait réagir l'huile neutre avec du méthanol, à une température comprise entre 220 et 250 °C, sous une pression de 40 à 100 bar et avec un rapport pondéral méthanol / huile de 1/3 à 2/1.

13. Procédé selon l'une des revendications 1 à 12, **caractérisé en ce que**, partant d'une huile acide, on met en oeuvre une étape préliminaire de réduction de sa teneur en acide gras par réaction avec de faibles quantités de glycérine, en présence d'un catalyseur hétérogène.

14. Procédé selon la revendication 13, **caractérisé en ce que** ledit catalyseur hétérogène est de même nature que celui utilisé dans l'étape (a).

## Patentansprüche

1. Verfahren zur gleichzeitigen Herstellung einer Zusammensetzung von Estern linearer Monocarboxylsäuren mit 6 bis 26 Kohlenstoffatomen und von Glycerin, von einem hohen Reinheitsgrad, **dadurch gekennzeichnet, dass** es umfasst:
- eine Stufe (a), in der man ein pflanzliches oder tierisches gegebenenfalls saures Öl mit einem Überschuss wenigstens eines aliphatischen Monoalkohols mit 1 bis 4 Kohlenstoffatomen in Gegenwart eines heterogenen Katalysators reagieren lässt, der unter den Aluminaten und den Silikaten von Zink, von Titan oder von Zinn gewählt ist, mit Entfernung des überschüssigen Monoalkohols und Trennung des Glycerins, wobei diese Stufe einen Rohester erzeugt, der Fettsäureester und restliche Monoglyceride enthält;
- eine Stufe (b), in der man den so erhaltenen Rohester einer Transesterifizierung oder Esterifizierungsreaktion von restlichen Monoglyceriden zu Di- und gegebenenfalls Triglyceriden in Gegenwart eines heterogenen Katalysators der gleichen Natur wie jenem bei Stufe (a) verwendeten durch Fettsäureester oder durch wenigstens eine Fettsäure mit 6 bis 26 Kohlenstoffatomen, die zugegeben ist, unterzieht; und
- eine Stufe (c), in der man zu einer Verdampfung der Esterzusammensetzung unter vermindertem Druck mit Rezyklierung des Verdampfungsrückstandes, der die Di- und gegebenenfalls die Triglyceride enthält, zu dem Ausgangsöl der Stufe (a) übergeht.

2. Verfahren nach Anspruch 1, **dadurch gekennzeichnet, dass** bei Stufe (a) und bei Stufe (b) als heterogener Katalysator ein Aluminat verwendet wird.

3. Verfahren nach Anspruch 2, **dadurch gekennzeichnet, dass** der heterogene Katalysator Zinkaluminat ist.

4. Verfahren nach einem der Ansprüche 1 bis 3, **dadurch gekennzeichnet, dass** man
- man in Stufe (a) ein neutrales Öl mit dem Monoalkohol bei einer Temperatur zwischen 180 und 250 °C unter einem Druck von 20 bis 100 bar und in Gegenwart von 2 bis 5 Gew.-% bezüglich des Öls von dem heterogenen Katalysator mit Entfernung durch Verdampfung des überschüssigen Monoalkohols und Trennung des Glycerins durch Dekantieren bei einer Temperatur von 20 bis 100°C reagieren lässt;
- man in der Stufe (b) die in dem Rohester enthaltenen Monoglyceride zu Di- und gegebenenfalls Triglyceriden, wobei die erhaltene Estermischung wenigstens 0,2% Monoglyceride enthält, durch Erhitzen bei einer Temperatur zwischen 180 und 250°C in Gegenwart des heterogenen Katalysators und mit Entfernung des bei der Reaktion gebildeten Monoalkohols umwandelt; und
- man bei der Stufe (c) den gebildeten Ester eines Monoalkohols unter einem Druck kleiner oder gleich 3 mm Quecksilber.

5. Verfahren nach einem der Ansprüche 1 bis 4, **dadurch gekennzeichnet, dass** die Temperatur der Stufen (a) und/oder (b) 180 bis 200°C ist.

6. Verfahren nach einem der Ansprüche 1 bis 5, **dadurch gekennzeichnet, dass** man bei einer Stufe (d) das in der Stufe (a) gebildete Glycerin reinigt.

7. Verfahren nach Anspruch 6, **dadurch gekennzeichnet, dass** man bei der Stufe (d) das Glycerin durch Durchgang über die Aktiverde und/oder Aktivkohle reinigt.

8. Verfahren nach einem der Ansprüche 1 bis 7, **dadurch gekennzeichnet, dass** man bei Stufe (b) in den Rohester aus der Stufe (a) wenigstens eine Fettsäure zugibt, die weder Phospholipide noch Alkalimetallionen enthält, derart, dass die Monoglyceride zu Di- und Triglyceriden mit Erzeugung von Wasser umgewandelt werden.

9. Verfahren nach einem der Ansprüche 1 bis 8, **dadurch gekennzeichnet, dass** das Ausgangsöl unter den Ölen von Soja, Raps, Sonnenblumen, Flachs, Erdnuss, Kopra, Kohl, Palme, Karthamin, Leinöl, Baumwolle, Talk, Schweineschmalz, Fischölen und den verbrauchten Frittierölen gewählt ist.

10. Verfahren nach einem der Ansprüche 1 bis 9, **dadurch gekennzeichnet, dass** der aliphatische Monoalkohol unter Methanol, Ethanol, sekundärem Butanol und Isobutanol gewählt ist.

11. Verfahren nach Anspruch 10, **dadurch gekennzeichnet, dass** der aliphatische Monoalkohol Methanol ist.

12. Verfahren nach Anspruch 11, **dadurch gekennzeichnet, dass** man bei Stufe (a) das neutrale Öl mit Methanol bei einer Temperatur zwischen 220 und 250°C und einem Druck von 40 bis 100 bar und mit einem Gewichtsverhältnis Methanol/ÖI von 1/3 bis 2/1 reagieren lässt.

13. Verfahren nach einem der Ansprüche 1 bis 12, **dadurch gekennzeichnet, dass** ausgehend von Ölsäure man eine Vorstufe zur Reduzierung von deren Gehalt an Fettsäure durch Reduktion mit geringen Mengen Glycerin in Gegenwart eines heterogenen Katalysators ausführt.

14. Verfahren nach Anspruch 13, **dadurch gekennzeichnet, dass** der heterogene Katalysator gleicher Natur wie jener in der Stufe (a) verwendete ist.

## Claims

1. A process for simultaneously producing a composition of esters of linear monocarboxylic acids containing 6 to 26 carbon atoms and glycerine with a high degree of purity, **characterized in that** it comprises:
• a step a) in which a plant or animal oil, which may or may not be acidic, is reacted with an excess of at least one aliphatic mono-alcohol containing 1 to 4 carbon atoms, in the presence of a heterogeneous catalyst, eliminating the excess mono-alcohol and separating the glycerine, this step producing a crude ester containing fatty acid esters and residual monoglycerides;
• a step b) in which the crude ester obtained undergoes transesterification or esterification of the residual monoglycerides to di- and possibly tri-glycerides, in the presence of a heterogeneous catalyst using the fatty acid esters or by at least one fatty acid containing 6 to 26 carbon atoms which is added; and
• a step c) in which the ester composition is evaporated under reduced pressure, recycling the evaporation residue containing the di- and possibly tri-glycerides to the starting oil of step a).

2. A process according to claim 1, **characterized in that**, in step a) and step b), an aluminate is used as the heterogeneous catalyst.

3. A process according to claim 2, **characterized in that** said heterogeneous catalyst is zinc aluminate.

4. A process according to any one of claims 1 to 3, **characterized in that**:
• in step a), a neutral oil is reacted with the mono-alcohol at a temperature in the range 180°C to 250°C, at a pressure of 20 to 100 bars and in the presence of 2% to 5% by weight with respect to the oil of the heterogeneous catalyst, with eliminating the excess mono-alcohol by evaporating and separating the glycerine by settling at a temperature of 20°C to 100°C;
• in step b), the mono-glycerides contained in the crude ester are transformed into di- and possibly tri-glycerides, the mixture of esters obtained containing less than 0.2% of mono-glycerides, by heating at a temperature in the range 180°C to 250°C, in the presence of the heterogeneous catalyst and with eliminating the mono-alcohol formed during the reaction; and
• in step c), the ester of the mono-alcohol which is formed is evaporated at a pressure of 3 mm of mercury or less.

5. A process according to any one of claims 1 to 4, **characterized in that** the temperature of steps a) and/or b) is 180°C to 200°C.

6. A process according to any one of claims 1 to 5, **characterized in that** in a step d), the glycerine formed in step a) is purified.

7. A process according to claim 6, **characterized in that** in step d), the glycerine is purified by passage over activated clay and/or activated charcoal.

8. A process according to any one of claims 1 to 7, **characterized in that** during step b), at least one fatty acid containing no phospholipids nor alkaline ions is added to the crude ester from step a) to transform the mono-glycerides to di- and tri-glycerides with the production of water.

9. A process according to any one of claims 1 to 8, **characterized in that** the starting oil is selected from soya oil, rapeseed oil, sunflower oil, linseed oil, peanut oil, coprah oil, palm nut oil, palm oil, safflower oil, linola oil, cottonseed oil, tallow, suet, fish oils and used cooking oil.

10. A process according to any one of claims 1 to 9, **characterized in that** the aliphatic mono-alcohol is selected from methanol, ethanol, secondary butanol and isobutanol.

11. A process according to claim 10, **characterized in that** said aliphatic mono-alcohol is methanol.

12. A process according to claim 11, **characterized in that** in step a), the neutral oil is reacted with methanol, at a temperature in the range 220°C to 250°C, at a pressure of 40 to 100 bars and with a methanol/oil weight ratio of 1/3 to 2/1.

13. A process according to any one of claims 1 to 12, **characterized in that** starting from an acidic oil, a preliminary step for reducing its fatty acid content by reaction with small quantities of glycerine is carried out in the presence of a heterogeneous catalyst.

14. A process according to claim 13, **characterized in that** said heterogeneous catalyst is of the same nature as that used in step a).
